# EUROPEAN PATENT APPLICATION

(11) **EP 1 348 767 A2**
(43) Date of publication of application: **01.10.2003**
(21) Application number: 03003924.2
(22) Date of filing: 21.02.2003
(51) Int. Cl.: C12Q 1/68, G01N 33/68

(54) **Polymer chip and method for identifying an ionic polymer**

(30) Priority: 28.03.2002 JP 2002090129
(71) Applicant: Hitachi Software Engineering Co., Ltd., Yokohama-shi, Kanagawa 230-0045 (JP)
(72) Inventor: Nishimura, Yoshifumi, Yokohama City University, Yokohama-shi, Kanagawa 230-0045 (JP); Nakao, Motonao, Hitachi Software Eng. Co., Ltd., Tokyo 140-0002 (JP); Morita, Toshiki, Hitachi Software Eng. Co., Ltd., Tokyo 140-0002 (JP)
(74) Representative: Liesegang, Roland, Dr.-Ing.

(57) **Abstract**

An ionic polymer that binds to an ionic polymer such as DNA is identified. In order to identify an ionic polymer that is positively charged, for example, another, negatively charged, polymer is immobilized on a substrate or a bead. A positively charged ionic polymer is adsorbed on the substrate or bead. When the surrounding ionic environment is suddenly decreased, the adsorbed complex is stabilized, whereas the structure of the immobilized, non-adsorbed ionic polymer becomes unstable. By providing the immobilized ionic polymer with a marker for differentiating a stable structure from an unstable one, it can be determined on which immobilized ionic polymer the ionic polymer to be identified has been adsorbed.

SEQUENCE LISTING

<110> Hitachi Software Engineering Co., Ltd.

<120> POLYMER CHIP AND METHOD FOR IDENTIFYING AN IONIC POLYMER

<130> PH-1700

<140>
<141>

<160> 4

<170> PatentIn Ver. 2.1

<210> 1
<211> 13
<212> DNA
<213> Artificial Sequence

<220>
<223> Description of Artificial Sequence:Synthetic DNA

<400> 1
gttagggtta ggg                                                    13

<210> 2
<211> 13
<212> DNA

<213> Artificial Sequence

<220>
<223> Description of Artificial Sequence:Synthetic DNA

<400> 2
cctaactgac acc                                                    13

<210> 3
<211> 13
<212> DNA
<213> Artificial Sequence

<220>
<223> Description of Artificial Sequence:Synthetic DNA

<400> 3
caatcccaat ccc                                                    13

<210> 4
<211> 13
<212> DNA
<213> Artificial Sequence

<220>
<223> Description of Artificial Sequence:Synthetic DNA

<400> 4

ggattgactg tgg 13

## Description

### BACKGROUND OF THE INVENTION

### 1. Technical Field

The present invention relates to a biochip for identifying ionic polymers such as represented by DNA and proteins, and a method of identifying ionic polymers using the biochip.

### 2. Background Art

There is an expectation that identifying DNAs and proteins can provide a tool for drug development and medical examinations.

A DNA is a polymer formed by a sequence of four kinds of bases, and two DNAs can specifically bind to one another through their sequences. Because the nucleic acids of both such DNAs are negatively charged, hybridization is carried out in a highly ionic solvent. The conventional biochips take advantage of this fact to detect a sample DNA by immobilizing a probe DNA on a substrate.

However, a DNA-binding protein, for example, cannot be identified by the conventional biochip. This is because a protein as a sample may be specifically adsorbed on a probe DNA on a biochip by its complementary DNA portion, and the protein may also be nonspecifically adsorbed on the probe DNA even though it is not supposed to, in light of the DNA. Because of such specific and nonspecific adsorptions that apparently occur on the probe DNA, it has been virtually impossible to identify sample proteins using the conventional biochip.

Thus, there is a need to develop a chip that can specifically bind to ionic polymers such as the multitude of proteins, and a technique of accurately identifying ionic polymers using the chip.

### SUMMARY OF THE INVENTION

The inventors arrived at the present invention after discovering that various ionic polymers such as DNA-binding proteins can be identified in a simple and accurate manner by using a polymer chip with a particular structure.

Specifically, the invention in one aspect provides a polymer chip for identifying an ionic polymer which comprises a substrate on which a first ionic polymer is immobilized, wherein a second ionic polymer having a marker is complementarily bound to the first ionic polymer as a probe, the polymer chip identifying an ionic polymer by having a third ionic polymer as a sample specifically bind thereto.

The first to third ionic polymers are preferably selected from proteins, polyamino acids, DNA, RNA and synthetic polymers, for example.

More specifically, the invention provides a DNA chip for identifying a DNA-binding protein, which comprises a substrate on which a first DNA is immobilized, wherein a second DNA having a marker is complementarily bound to the first DNA.

Preferably, the number of bases in the base sequence of the first DNA and second DNA is from four to 13, respectively. When the number of bases is in this range, the complementary binding of the first and second DNAs is strengthened, and further, the specific binding of the DNA chip comprising these DNAs and a sample DNA-binding protein based on their chemical and physical structures is strengthened, thus making the identification of the sample protein easier and more accurate.

The material and shape of the substrate are not particularly limited, and they can be selected from the materials and shapes that are employed in the substrates of biochips. Preferably, the material is selected from glass, silica gel, polystyrene, polypropylene and membrane, and the substrate is either plate- or bead-shaped.

The marker attached to the second ionic polymer is selected from ones used in the field of biochip technologies. Particularly, substances that emit fluorescence are preferable for the optical processes in a readout step.

In another aspect, the invention provides a method of identifying an ionic polymer which comprises the steps of providing a probe by complementarily binding a second ionic polymer having a marker to a first ionic polymer in an ionic solvent, wherein the first ionic polymer is immobilized on a substrate in a polymer chip, drying the probe, having a third ionic polymer as a sample adsorbed on the probe, washing the probe with an ionic solvent to remove the third ionic polymer that is nonspecifically bound (i.e., one that binds to the probe even though it should not), washing the probe with a nonionic solvent to remove the second ionic polymer having a marker except for a part where the third ionic polymer is specifically bound (when it should be), and reading the amount of the marker that remains.

In the case where the probe in which the second ionic polymer having a marker is complementarily bound to the first ionic polymer has the same charge as that of the sample third ionic polymer, preferably a step is provided in which a fourth sample polymer having an opposite ionicity is added to firmly bind the probe and the sample third ionic polymer, and the probe is then washed with a nonionic solvent.

The first to third ionic polymers are preferably selected from proteins, polyamino acids, DNA, RNA and synthetic polymers, for example.

More specifically, the invention identifies a sample protein by providing a probe by complementarily binding a second DNA having a marker to a first DNA in an ionic solvent, wherein the first DNA is immobilized on a substrate in a DNA chip, drying the probe, having a DNA-binding protein as a sample adsorbed on the probe, washing the probe with an ionic solvent to remove the protein that is nonspecifically bound, washing the probe with a nonionic solvent to remove the second DNA having a marker except for a part where the DNA-binding protein is specifically bound, and reading the amount of the remaining marker.

Preferably, the number of bases in the base sequence of the first DNA and second DNA is from four to 13, respectively. When the number of bases in the base sequence is in this range, the complementary binding of the first and second DNAs is strengthened, and further, the specific binding of the DNA chip comprising these DNAs and a sample DNA-binding protein based on their chemical and physical structures is strengthened, thus making the identification of the sample protein easier and more accurate.

The material and shape of the substrate are not particularly limited, and they can be selected from the materials and shapes that are employed in the substrates of biochips. Preferably, the material is selected from glass, silica gel, polystyrene, polypropylene and membrane, and the substrate is either plate- or bead-shaped.

The marker attached to the second ionic polymer is selected from ones used in the field of biochip technologies. Particularly, a substance that emits fluorescence is preferable for the optical processes in a readout step.

The kind of the non-ionic solvent is not particularly limited. Examples include one selected from the group containing pure water, alcohol, acetone, and hexane, or a mixture of two or more substances therein. The ionic solvent is not particularly limited either, and examples include an aqueous solution of sodium chloride and an aqueous solution of potassium chloride.

Biopolymers such as DNA and proteins (polyamino acids and polypeptides) are charged. DNA has a negative charge due to a phosphate group. Proteins have either a positive or negative charge, depending on the kind of the amino acids. Double strands of DNA normally repel each other due to negative ions and they cannot be hybridized in pure water. In a system in which positive ions exist, however, they can be hybridized by hydrogen bonding in the presence of ions when, for example, an Na⁺ ion forms a chelate with a phosphate group of DNA. Proteins having a positive charge, for example, can bind to DNA having a negative charge via the charged portions.

When a biochip in which these biopolymers are bonded by their charges is washed with a solvent in which the ion intensity is varied, at low ion intensities polymers having the same charge are dissociated and polymers having opposite charges are firmly bound. At high ion intensities, even the polymers having the same charges can be bound while the bond of polymers having different charges becomes weak and they are dissociated.

By thus varying the ion intensity, various polymers can be identified.

The DNA-binding proteins used in the present invention are those proteins that have an affinity for DNA and specifically or nonspecifically bind to a base sequence. Examples of such DNA-binding proteins include: 1) double-stranded DNA-binding proteins that control gene expression by introducing changes in the DNA structure; 2) single-stranded DNA-binding (SSB) proteins that are indispensable for the processes of DNA replication, recombination and repair; 3) proteins involved in the maintenance of higher structures of chromosomes; 4) DNA-dependent ATPase; and 5) topoisomerases that form DNA superhelix structures. Many of the proteins 1) are transcription factors, for which several structural motifs are known, such as a helix-turn-helix that was found in the structure of a lambda phage Cro protein and a cAMP receptor protein; a zinc finger in which a zinc ion is chelated by cysteines and histidines, and; a leucine zipper that is formed when two protein molecules each having a leucine arranged on a side of α-helix are combined as in a zipper. The proteins 2), namely the SSB proteins, are found in organisms from bacteriophages to higher forms of life. The proteins 3) are represented by a histone protein in the chromosomes of eukaryotes, and they form nucleosome structures. In bacterium too, similar HU proteins bind to form a nucleosome-like structure. The proteins 4) include helicases that promote DNA replication (DnaB protein, for example), recombination (RecA, RecBC proteins), and unwinding of double-stranded DNA. Furthermore, in the present invention, not only the above-mentioned proteins that originally have a DNA-binding property but also proteins that have come to possess a DNA-binding property by physical or chemical processing can be used.

Thus, in the invention, this wide variety of DNA-binding proteins can be used. The invention includes not only the case of using a single kind of DNA-binding protein but also the case of using two or more kinds of DNA-binding proteins in combination.

DNA-binding proteins have a positive charge at the DNA-binding domains, and they are characterized by their specific or nonspecific binding to DNA or RNA having a negative charge. In a double-stranded DNA to which these DNA-binding proteins have bound, the bond becomes stronger the lower the ion intensity.

### BRIEF DESCRIPTION OF THE DRAWINGS

Fig. 1 schematically shows a protein-identifying DNA chip as an example of the polymer chip for identifying an ionic polymer according to the invention.
Fig. 2 illustrates the process of identifying a protein using the protein-identifying DNA chip of Fig. 1.
Fig. 3 shows a biochip.
Fig. 4 shows images after DNA hybridization.
Fig. 5 is an image after addition of a TRF protein.
Fig. 6 is an image after addition of a c-Myb protein.
Fig. 7 is an image after addition of a Pur protein.
Fig. 8 is a graph in which the individual images are numerically represented and compared.

### DESCRIPTION OF THE INVENTION

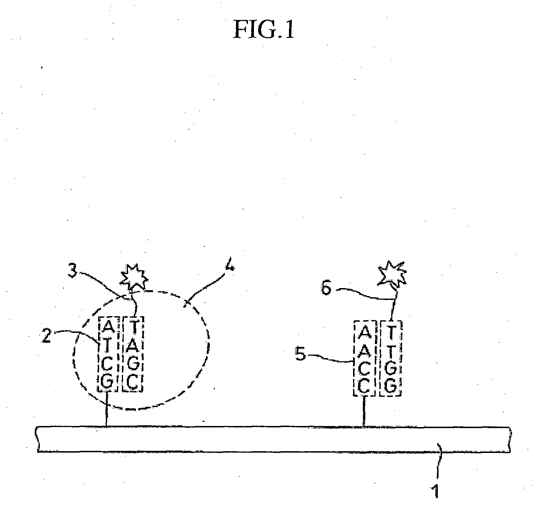

Fig. 1 schematically shows a protein-identifying DNA chip as an example of the polymer chip for identifying an ionic polymer according to the invention. It comprises a substrate 1 on which a first DNA 2 having a base sequence of GCTA is immobilized. When a second DNA 3 labeled by a fluorescent substance and having a base sequence of CGAT is added to an ionic solvent in which the DNA chip is placed, they complementarily bind to form a double strand. Likewise, when a different second DNA 6 labeled by another fluorescent substance and having a base sequence of GGTT is added thereto, it complementarily binds to a DNA 5 having a base sequence of CCAA to form a double strand. When an unidentified protein 4 is added to the DNA chip, the protein 4 binds to the double strand in which DNA 2 and DNA 3 are complementarily bound. However, this protein 4 does not bind to the double strand in which DNA 5 and DNA 6 are complementarily bound. By measuring such a phenomenon via fluorescence intensity, the protein can be identified.

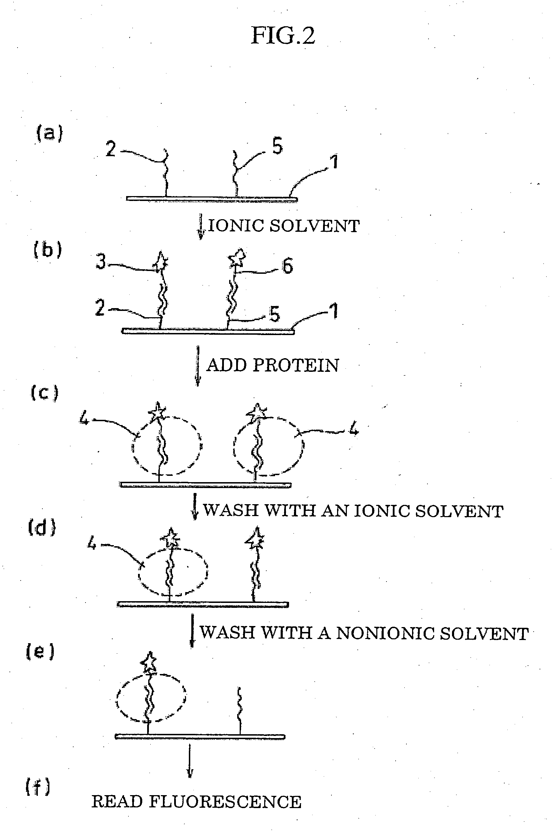

Fig. 2 illustrates the process of identifying a protein using the protein identifying DNA chip of Fig. 1. In step (a), a DNA chip is prepared in which DNA 2 and DNA 5 are immobilized on a substrate 1. In step (b), DNA 3 and DNA 6 that are labeled by a fluorescent substance are complementarily bound on the DNA chip in the presence of an ionic solvent. Then, the ionic solvent is removed and an unidentified protein 4 is added. In step (c), the protein 4 not only binds specifically to the double strand of DNA 2 and DNA 3, as it should, but also binds nonspecifically to the double strand of DNA 5 and DNA 6, which in essence it should not. Thus, in step (d), the DNA chip is washed with an ionic solvent to remove the protein that has bound nonspecifically. The DNA chip is further washed in step (e) with a nonionic solvent to remove DNA 6 labeled by a fluorescent substance. In step (f), the remaining fluorescence is measured and the protein is identified based on the measurement.

### Examples

Hereafter, examples of the invention will be described, in which proteins TRF, c-Myb, and Pur that had already been identified were used as samples. TRF is described by Nishikawa, T., Nagadoi, A., Yoshimura, S., Aimoto, S., and Nishimura, Y. in "Solution structure of the DNA-binding domain of human telomeric protein, hTRF1," Structure, 6, pp. 1057-1065 (1998). C-Myb is described by Ogata, K., Hojo, H., Aimoto, S., Nakai, T., Nakamura, H., Sarai, A., Ishii, S. and Nishimura, Y. in "A Helix-turn-helix-related motif with conserved tryptophans forming a hydrophobic core," Proc. Nat. Acad. Sci. USA, 89, pp. 6428-6432 (1992). Pur is described by Nagadoi, A., Morikawa, S., Nakamura, H., Enari, M., Kobayashi, K., Yamamoto, H., Sampei, G., Mizobuchi, K., Schumacher, M. A., Brennan, R.G., and Nishimura, Y. in "Structural comparison of the free and DNA-bound forms of the purine repressor DNA-binding domain," Structure 3, pp. 1217-1224 (1995).

Initially, a biochip was produced as follows.

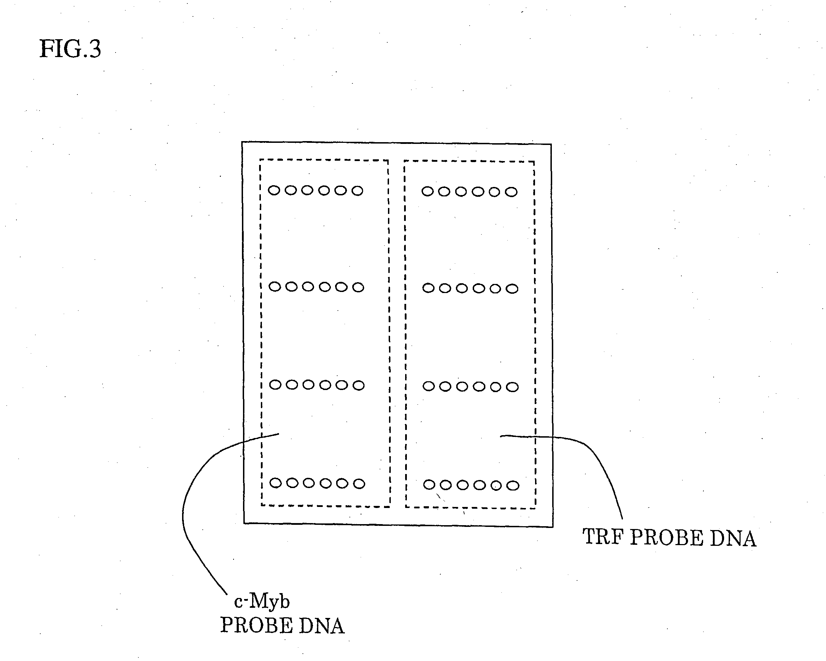

A TRF probe DNA with a sequence of 5'-GTTAGGGTTAGGG-3' and a c-Myb probe DNA with a sequence of 5'-CCTAACTGACACC-3' were biotinylated at their 5'-ends and spotted at 24 locations on an avidin-coated slide glass by SPBIO (from Hitachi Software Engineering Co., Ltd). The spotted probe DNAs were adjusted to a concentration of 1 mM by adding pure water. Fig. 3 illustrates the spotted biochip.

Two strands of sample DNA were prepared. A TRF sample DNA had a sequence of 3'-CAATCCCAATCCC-5' and a c-Myb sample DNA had a sequence of 3'-GGATTGACTGTGG-5'. They were both labeled with the fluorescent pigment Cy5 at their 5'-ends.

After adjusting the concentration of the sample DNAs to 10 µM in a 5x SSC solution, 20 µL of each sample was added onto the prepared biochip and hybridized for 30 min in a humid environment to prevent drying.

After hybridization, the biochip was washed with 1x SSC and dried, and then read by a fluorescence scanner CRBIO2 (from Hitachi Software Engineering Co., Ltd).

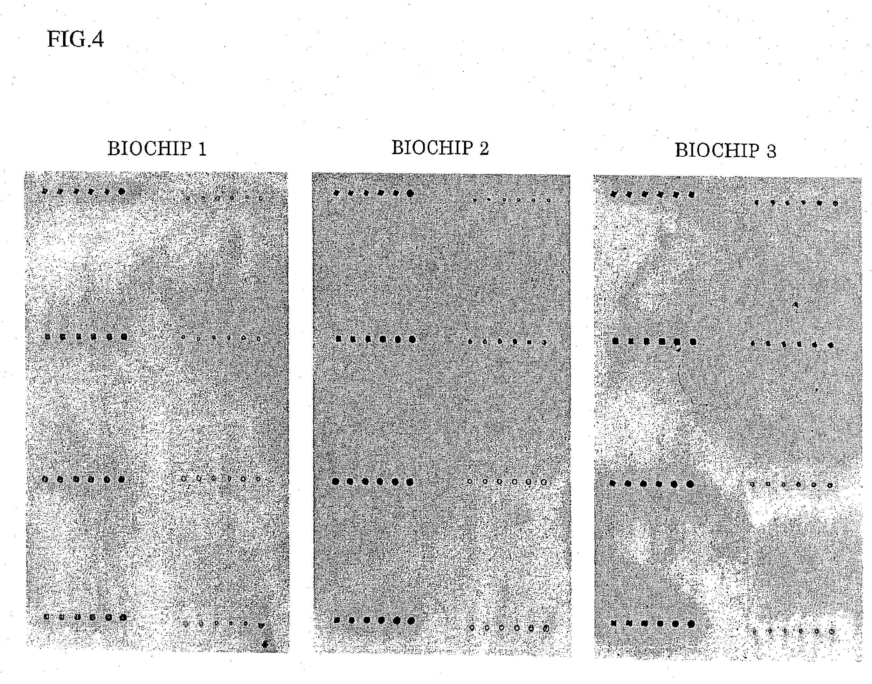

Fig. 4 shows images that were read. Darkened spots indicate the locations where the Cy5-labeled samples are present. Since DNA hybridizes with a complementary strand, the TRF sample DNA is hybridized where the TRF probe DNA was spotted and the c-Myb sample DNA is hybridized where the c-Myb probe DNA was spotted. This has been confirmed by the result of hybridizing the sample DNAs individually (images are not shown).

Thereafter, a TRF protein sample liquid prepared from TRF protein at 110 µM, KPB (phosphoric acid) at 5 mM, and NaCl at 30 mM was added onto the hybridized biochip and hybridized for 10 min.

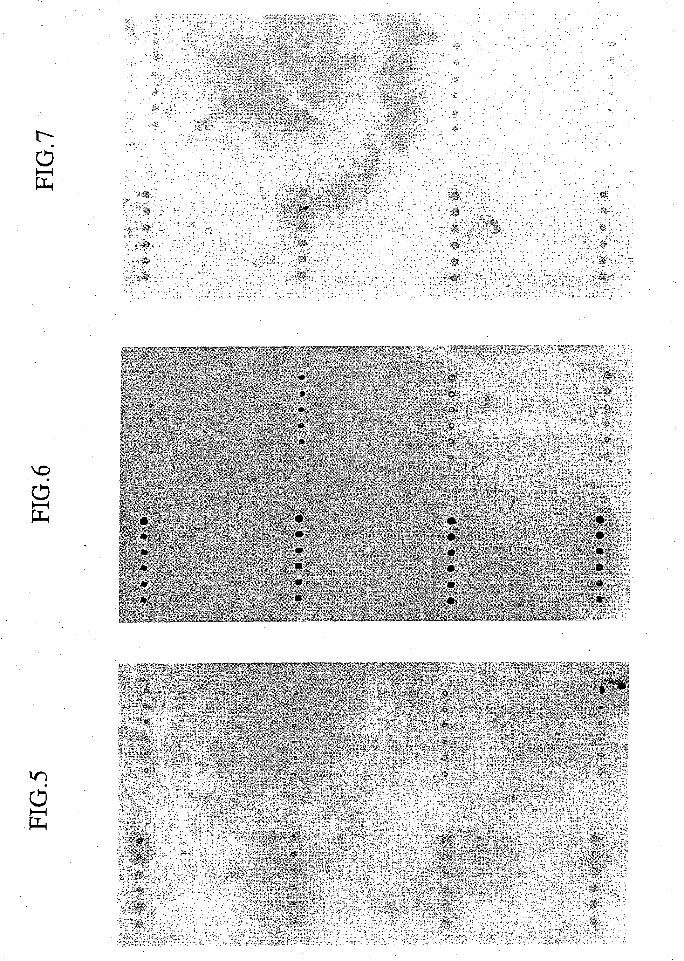

After washing the biochip with 1x SSC at 40°C and then with pure water at 40°C, the biochip was read by CRBIO2 (from Hitachi Software Engineering Co., Ltd). The image of the individual spots that were read is shown in Fig. 5.

Similarly, c-Myb protein at 74 µM, KPB at 10 mM, DTT at 1 mM, and NaN₃ at 0.1 mM were hybridized onto the biochip 2. The biochip 2 was then washed with 1x SSC and pure water and read by CRBIO2. The image of the individual spots that were read is shown in Fig. 6.

Similarly, Pur protein at 527 µM, KPB at 1 mM, KCl at 20 mM, and NaN₃ at 0.05 mM were hybridized on the biochip 3. The biochip 3 was washed with 1x SSC and pure water, and then read by CRBIO2 (from Hitachi Software Engineering Co., Ltd). The image of the individual spots that were read is shown in Fig. 7.

It will be seen from Figs. 5, 6 and 7 that when DNA that recognizes the respective proteins is present, each protein strongly binds to the double-stranded DNA to which it hybridizes, so that detection can be made even in pure water without dissociation of double strands.

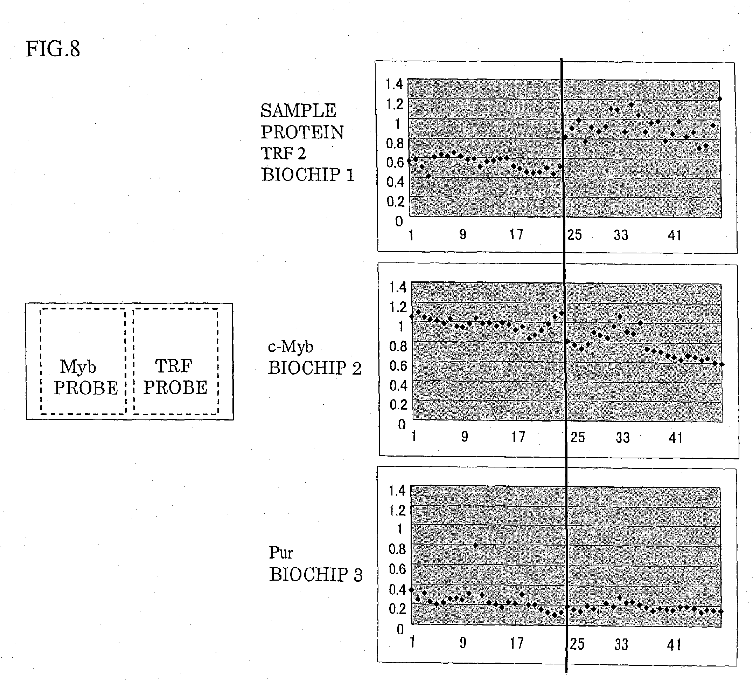

In Fig. 8, the fluorescence intensities at the spot locations in the images of Figs. 5 to 7 are numerically represented relative to the fluorescence intensities at the spot locations in the images of Fig. 4. In this figure, the vertical axis indicates the relative fluorescence intensities, while the horizontal axis indicates the spot number. Spot numbers 1-24 relate to the fluorescence intensity comparison by the c-Myb probe, and spot numbers 25-48 relate to the fluorescence intensity comparison by the TRF probe.

Ideally, the spots where there is no decrease in fluorescence intensity have the value of 1.0 and values less than 1.0 are assigned as the fluorescence intensity decreases. It will be seen from Fig. 8 that in the case of the TRF probe spots, the decrease in fluorescence intensity is suppressed by adding the TRF protein, while in the case of the c-Myb probe spots, the decrease is suppressed by adding the c-Myb protein. These results indicate that each protein firmly bound to the double-stranded DNA to which it hybridized and the double-stranded DNA remained bound without dissolution even in pure water, in which there are no ions.

Thus, the present invention can effectively and comprehensively identify ionic polymers such as transfer factor proteins. In particular, the invention can identify a number of proteins easily and accurately. The invention can thus provide a tool for drug development and medical examinations.

## Claims

1. A polymer chip for identifying a third ionic polymer as a sample, comprising a substrate on which a first ionic polymer is immobilized, wherein a second ionic polymer having a marker binds as a probe to the first ionic polymer by complementary bonding.

2. The polymer chip for identifying an ionic polymer according to claim 1, wherein the first to third ionic polymers are selected from proteins, polyamino acids, DNA, RNA, and synthetic polymers.

3. The polymer according to claim 1 or 2, wherein the first ionic polymer is a first DNA, the second ionic polymer is a second DNA and the third ionic polymer is a DNA-binding protein.

4. The protein-identifying DNA chip according to claim 3, wherein the number of bases in the base sequence of the first and second DNA is from 4 to 13, respectively.

5. The polymer chip according to any one of claims 1 to 4, wherein the material of the substrate is selected from glass, silica gel, polystyrene, polypropylene, and membrane.

6. The polymer chip according to any one of claims 1 to 5, wherein the substrate is plate- or bead-shaped.

7. The polymer chip according to any one of claims 1 to 6, wherein the marker is a fluorescent substance.

8. A method of identifying an ionic polymer, comprising the steps of:
providing a probe by binding, in an ionic solvent, a second ionic polymer having a marker to a first ionic polymer immobilized on a substrate in a polymer chip by complementary binding; drying the probe;
having a third ionic polymer as a sample absorbed on the probe;
washing the probe with an ionic solvent to remove the third ionic polymer that is nonspecifically bound;
washing the probe with a nonionic solvent to remove the second ionic polymer having a marker except for a part where the third ionic polymer is specifically bound; and
reading the amount of the remaining marker.

9. The method of identifying an ionic polymer according to claim 8, wherein, when the probe, in which the second ionic polymer having a marker is bonded to the first ionic polymer by complementary bonding, has the same charge as that of the third ionic polymer as a sample, a fourth sample polymer with an opposite ionicity is added in order to firmly bind the probe and the third ionic polymer, followed by washing the probe with a nonionic solvent.

10. The method of identifying an ionic polymer according to claim 8 or 9, wherein the first to third ionic polymers are selected from proteins, polyamino acids, DNA, RNA, and synthetic polymers.

11. The method according to any one of claims 8 to 10, wherein the first ionic polymer is a first DNA, the second ionic polymer is a second DNA, the third ionic polymer is a DNA-binding protein and the polymer chip is a DNA-chip.

12. The method according to claim 11, wherein the number of bases in the base sequence of the first and second DNA is from 4 to 13, respectively.

13. The method according to any one of claims 8 to 12, wherein the material of the substrate is selected from glass, silica gel, polystyrene, polypropylene, and membrane.

14. The method according to any one of claims 8 to 13, wherein the substrate is plate- or bead-shaped.

15. The method according to any one of claims 8 to 14, wherein the marker is a fluorescent substance.

16. The method according to any one of claims 8 t o15, wherein the nonionic solvent is one selected from the group containing pure water, alcohol, acetone, and hexane, or a mixture of two or more substances therein.

17. The method according to any one of claims 8 to 16, wherein the ionic solvent is an aqueous solution of sodium chloride or an aqueous solution of potassium chloride.
